# EUROPEAN PATENT APPLICATION

(11) **EP 1 974 744 A1**
(43) Date of publication of application: **01.10.2008**
(21) Application number: 07023887.8
(22) Date of filing: 15.06.2001
(51) Int. Cl.: A61K 39/12, A61K 39/29, A61P 31/14, C12N 15/82, C07K 14/10, A01H 5/00

(54) **Immunization through oral administration of a vaccine with an edible product**

(30) Priority: 16.06.2000 US 596060
(62) Divisional of application: 01943760.7
(71) Applicant: HADASIT MEDICAL RESEARCH SERVICES AND DEVELOPMENT LTD., 91120 Jerusalem (IL)
(72) Inventor: Gilad, Mali Kezinel, 96410 jerusalem (IL); Galun, Esra, 76100 Rehovot (IL); Mitchell, Leslie, 99880 Moshav Bar Giyyora (IL); Giladi, Hilla, Mevaseret Zion (IL); Gauss-Mueller, Verena, 23562 Lubeck (DE); Galun, Eithan, Har Adar (IL)
(74) Representative: Wibbelmann, Jobst

(57) **Abstract**

A vaccine produced in edible plant and/or animal products, as well as a method of second generation vaccine development through the production of at least one complete structure of a pathogen in a transgenic plant or animal. Preferably, the present invention enables the production of virus-like particles in edible food plants, through the co-expression of a plurality of proteins and/or of a plurality of portions of such proteins. The co-expression of viral structural proteins should enhance the proper presentation of viral related antigens to the human immune system.

## Description

This is a divisional application of European patent application 01 943 760.7 filed on June 15, 2001 (published on March 12, 2003; publication no. EP 1 289 547 A); the disclosure of which (claims, description, and drawings) is incorporated herewith by reference in its entirety.

### FIELD OF THE INVENTION

The present invention relates to a method of immunizing a subject against a disease-causing pathogen through administration of a vaccine with an edible product, and in particular, to such a method in which the vaccine is administered through a genetically modified plant product. Preferably, the vaccine is directed against the Hepatitis A virus (HAV).

### BACKGROUND OF THE INVENTION

Infectious diseases are a worldwide cause of morbidity and mortality. Certain of these particularly affect individuals with weaker immune systems such as very young children, and are the primary cause of childhood mortality on a worldwide basis (The World Health Report 1997, WHO). Travel and other contact between populations have increased the risk for spread of bacterial and viral pathogens, thereby demanding a higher degree of community protection than ever before. The most efficient and effective strategy for preventing these diseases is by mass vaccination (Review: Vaccine supplement. Nature Medicine, 4:474-534, 1998). However, despite significant progress in biotechnological engineering and technological advancements in mass production of vaccines, these remain costly and often, unavailable on a practical basis. Currently, most vaccines are administered parenterally by injection. Further, such vaccines must be kept refrigerated until administered. Thus, mass vaccination programs currently require a large supply of vaccine, maintenance of the cold chain, and supplies of sterile syringes and needles. These requirements render such programs difficult or impossible to perform in some jurisdictions, particularly in Third World countries.

Hepatitis A virus (HAV), a member of the genus *Hepatovirus* within the viral family *picornaviriade,* is one example of a pathogen for which the vaccine is currently difficult to administer through such mass vaccination programs. The hepatitis A virus (HAV) is one of the many viral diseases transmitted by the fecal-oral route, and is an example of a disease which is associated with early childhood morbidity and mortality in many countries. After entering the body through the gastrointestinal system orally, HAV migrates to the liver for tissue specific replication leading to clinical hepatitis. No mass vaccination program for HAV has been undertaken.

HAV vaccine is currently generated in primate tissue cultures, which produce a low viral titer, and is therefore expensive. The expense and difficulty of production are such that at present the HAV vaccine can only be purchased for small-scale programs. Hence, new solutions for cheap and effective mass vaccination are needed. Providing a vaccine through a simple method could significantly increase vaccine uptake and population protection against HAV.

Recently, it has been demonstrated that vaccination against bacteria, such as salmonella, or viral pathogens, such as HIV, may possibly be enhanced through the rectal administration of attenuated or killed bacteria/viruses (see for example "Oral and rectal immunization of adult female volunteers with a recombinant attenuated Salmonella typhi vaccine strain"; Nardelli-Haefliger D, Kraehenbuhl JP, Curtiss R 3rd, Schodel F, Potts A, Kelly S, De Grandi P. Infect Immun. 1996;64:5219-24; and "A rational basis for mucosal vaccination against HIV infection"; Lehner T, Bergmeier L, Wang Y, Tao L, Mitchell E. Immunol Rev. 1999 170:183-96). Rectal, nasal or oral administrations of vaccines have been shown to elicit both humoral and cellular immune responses. However, these results have been variable. Nevertheless, oral polio vaccine (of Sabin) has been highly successful in generating protective virus-neutralizing antibodies.

Unfortunately, no such method for administering the HAV vaccine has been successful through a route of administration other than injection. Therefore, the HAV vaccine is currently difficult to administer, such that any mass vaccination program would be expensive and complicated to perform.

Many other vaccines would also be most usefully administered orally. Even for those vaccines which are available in an orally-administered form, however, mass vaccination programs are difficult to perform because of storage and handling requirements for the vaccines, and of course the cost of vaccination. Currently, vaccines are typically produced from various tissue and/or bacterial cell cultures, which is an expensive and difficult production method. In addition, the resultant vaccine must be handled carefully, often requiring refrigeration and/or other types of special handling which may be difficult or impossible to perform in more remote or less technologically developed areas.

Recent investigations have produced genetically engineered plants capable of expressing bacterial or viral proteins, in an attempt to overcome the previously described problems of mass production and storage of vaccines. For example, cholera toxin B subunit oligomers were expressed in transgenic potato plants, although the resultant plants were not tested for immunogenicity (Arakawa et al., "Expression of Cholera Toxin B Subunit Oligomers in Transgenic Potato Plants"; Transgenic Research, 6:403-413; 1997). Similarly, the rabies virus glycoprotein, which coats the outer surface of the virus, has also been expressed in transgenic tomato plants, although again the resultant plants were not tested for immunogenicity (McGarvey et al., "Expression of the Rabies Virus Glycoprotein in Transgenic Tomatoes"; Biotechnology, 13:1484-1487, 1995). Also, US Patent No. 5,914,123 describes vaccines which are expressed in plants, but also does not provide any data concerning the immunogenicity of the resultant plant matter vaccine. US Patent Nos. 5,612,487 and 5,484,719 also describe anti-viral vaccines which are expressed in plants, but similarly do not provide any data concerning the immunogenicity of the resultant plant matter vaccine.

Not all plant-based vaccines which have been tested have proven to be immunogenic. Furthermore, consumption of some vaccine-containing plants as food, that is, through normal oral consumption of the plant product, only triggered a partially protective immune response in animals and humans [Sandhu et al, "Oral immunization of mice with transgenic tomato fruit expressing respiratory syncytial virus-F protein induces a systemic immune response", Transgenic Research, 9: 127-135, 2000; Richter et al., "Production of hepatitis B surface antigen in transgenic plants for oral immunization", Nature Biotechnology, 18:1167-1171, 2000; and Mason et al., "Expression of Norwalk virus capsid protein in transgenic tobacco and potato and its oral immunogenicity in mice", PNAS, 93:5335-5340, 1996].

A partial explanation for the low immunogenicity of these plant-based vaccines, particularly for certain pathogens, relates to the fact that the targeted antigens have been expressed as isolated proteins or peptides, or even as only portions of proteins, and not in the context of the intact pathogen. Hence these proteins cannot assume the correct tertiary structure, recognized by the immune system. For example, the HAV capsid proteins expressed individually do not elicit neutralizing antibodies. This result is not surprising, as earlier studies showed that other viruses and bacterium exhibit similar behavior when only portions of the overall structure are administered as conventional vaccines [Almond and Heeney, "AIDS vaccine development in primate models", AIDS 12(suppl A):S133-140, 1998; Mayr et al., "Development of replication-defective adenovirus serotope 5 containing the capsid and 3C protease coding regions of Foot-and-Mouth Disease virus as a vaccine candidate", Virology, 263:496-506, 1999; and Wigdorovitz et al., "Induction of a protective antibody response to Foot-and-Mouth Disease virus in mice following oral or parenteral immunization with alfalfa transgenic plants expressing the viral structural protein VP1", Virology, 2553:347-353,1999].

### SUMMARY OF THE INVENTION

The background art does not teach or suggest a vaccine which is produced by edible plant and/or animal materials, for regular consumption (that is, through oral administration), which contains at least one complete structure of a disease-causing pathogen. The background art also does not teach or suggest that such a complete structure may optionally be a plurality of proteins, or alternatively may comprise a single molecule which mimics the structure of pathogen. The background art also does not teach or suggest a successfully immunogenic vaccine for viruses such as HAV. Also, the background art does not teach or suggest a method for producing such vaccines in transgenic plants and/or animals.

There is thus a need for, and it would be useful to have, a vaccine which is able to successfully elicit a protective immune response to disease-causing pathogens, through the production of at least one complete structure of the pathogen, regardless of whether the pathogen is viral, bacterial or parasitic in nature.

The present invention overcomes these problems of the background art, and also provides a solution to a long-felt need for producing vaccines in edible plant and/or animal products, by providing a new method of second generation vaccine development through the production of at least one complete structure of a pathogen in a transgenic plant or animal, as well as by providing the vaccines themselves. Preferably, the present invention enables the production of virus-like particles in edible food plants, through the co-expression of a plurality of proteins and/or of a plurality of portions of such proteins as recombinant peptide structures. The co-expression of viral structural proteins should enhance the proper presentation of viral related antigens to the human immune system

As a preferred example of the operation of the present invention, a vaccine was developed for hepatitis A virus (HAV). Previous attempts of vaccination with isolated HAV capsid proteins failed to elicit a protective immune response, because neutralizing antibodies recognize specific structures on the viral particle which are created only after the assembly of the capsid. To overcome this problem, preferably the present invention includes the construction of two HAV plasmids carrying a non-infectious HAV genome lacking the 5'UTR, for stable transformation of plants. In one plasmid (pGPPatΩHAV) transcription of HAV is driven from the patatin promoter for expression in tomato fruits, and in the second plasmid (pJDHAV) transcription is under the control of the 35SCaMV promoter and should be expressed in the green parts of transgenic plants. For the assessment of the immune response to the HAV transgenes the crops of transgenic plants are consumed by experimental animals.

According to preferred embodiments of the present invention, the technology which is developed for vaccines can also optionally and more preferably be applied for gene targeting to specific organs through the consumption of edible plant components containing none infectious viral particles as gene vehicles.

As a model disease, a method for administering a regular HAV vaccine to a subject by absorption through a mucosal tissue is also provided, particularly through the mucosa of the rectum. This method enables the HAV vaccine to be administered to the subject rectally, for example as a suppository or other rectal dosage form, and to successfully immunize the subject against HAV. Thus, the methods of present invention overcome problems of background art methods of administration, such as parenteral administration which is difficult and expensive to perform.

Hereinafter, the digestive system is defined as mouth, esophagus, stomach, small intestine, large intestine and rectum, or any portion thereof.

### BRIEF DESCRIPTION OF THE DRAWINGS

The foregoing and other objects, aspects and advantages will be better understood from the following detailed description of a preferred embodiment of the invention with reference to the drawings, wherein:

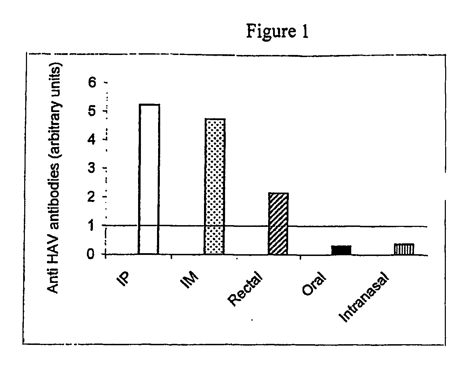

FIG. 1 is a graph of the titer of experimental mice after administration of the HAV vaccine; six Balb/c mice were administered 20µl nasally, 100 µl rectally or 100 µl into the peritoneum (IP) of a commercial preparation of the HAV vaccine (HAVRIX™). The vaccine was administered twice, at days 0 and 21, and tested for anti-HAV antibodies (HAVAB, EIA for total anti-HAV antibodies, Abbott) 35 days after the first vaccination.

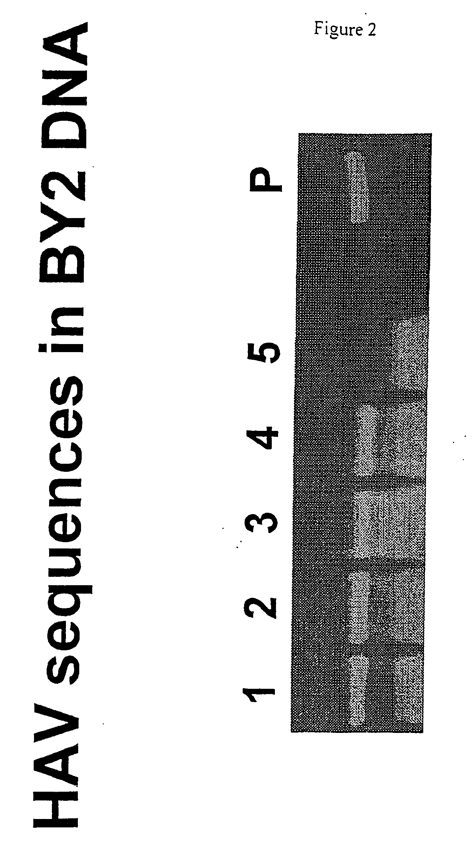

FIG. 2 shows the presence of HAV sequences in the transfected cells (lane 1-4, lane 5 negative control and lane P positive control) was determined by PCR using primers from the VP1-VP3 viral structural region of the virus for cells transfected with pG35SΩHAV, containing the entire HAV coding sequences under the control of the fruit-specific patatin promoter and the omega enhancer sequence.

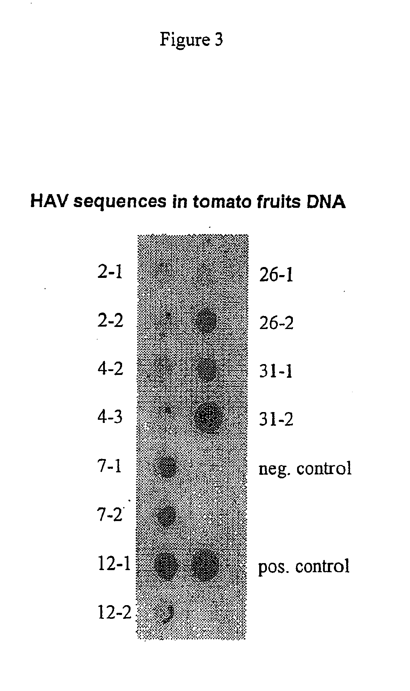

FIG. 3 depicts a dot blot and hybridization result with radioactive labeled HAV cDNA probe of DNA produced from 12 transgenic tomato plant leaves. As seen in the figure all the 12 plants contained HAV DNA sequences.

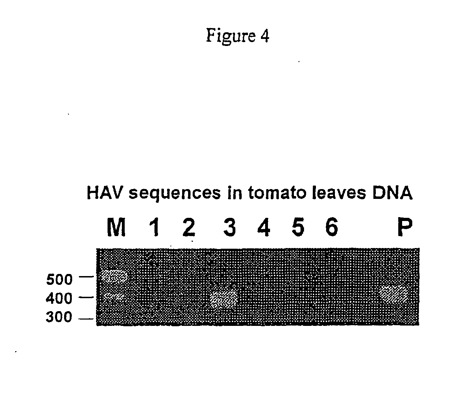

FIG. 4 shows results of assessing part of the plants of Figure 3 by PCR. Abbreviations are as follows: M, molecular weight size marker; 1-6, tomato leaves DNA; P, positive control.

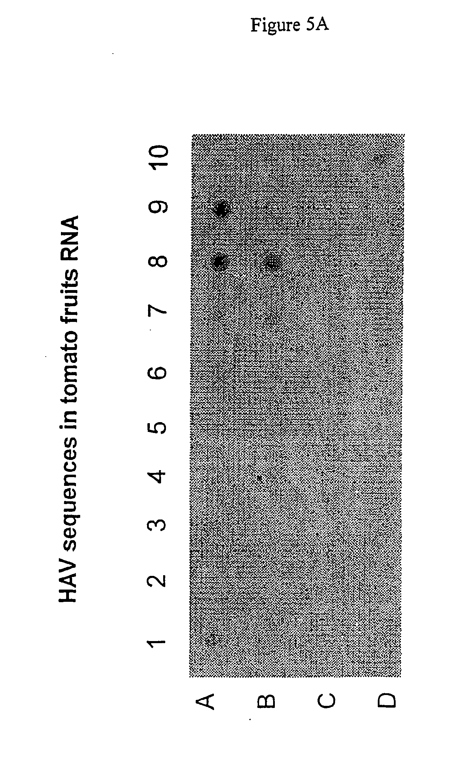

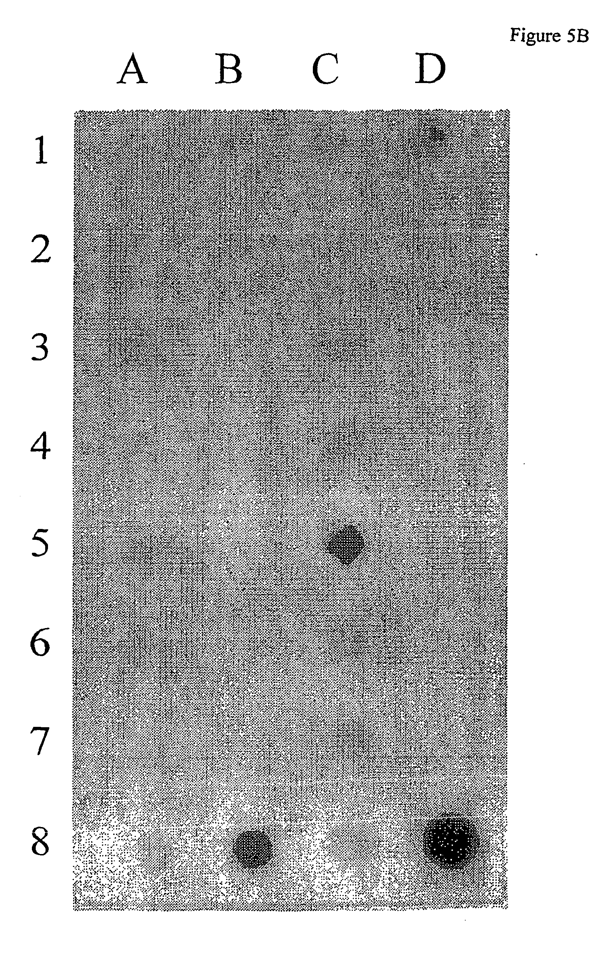

Figure 5A shows an example of RNA extracted from 17 plants that were analyzed in a similar fashion. In order to eliminate the possibility that the positive signal seen was due to residual DNA in the RNA samples, the RNA extracted from the tomato fruits with DNase I and the dot blot analysis was repeated (Figure 5B). Abbreviations are as follows: (Figure 5A) A1 - B10, tomato fruits RNA; D1 & D3, negative control; D10, positive control. (Figure 5B): A1-8, B1-2,C1-8,D1-2, tomato fruits RNA; B-3, 100 pg HAV plasmid treated with DNase I, B8 and D8, 100 and 200 pg respectively of HAV plasmid not treated with DNase I.

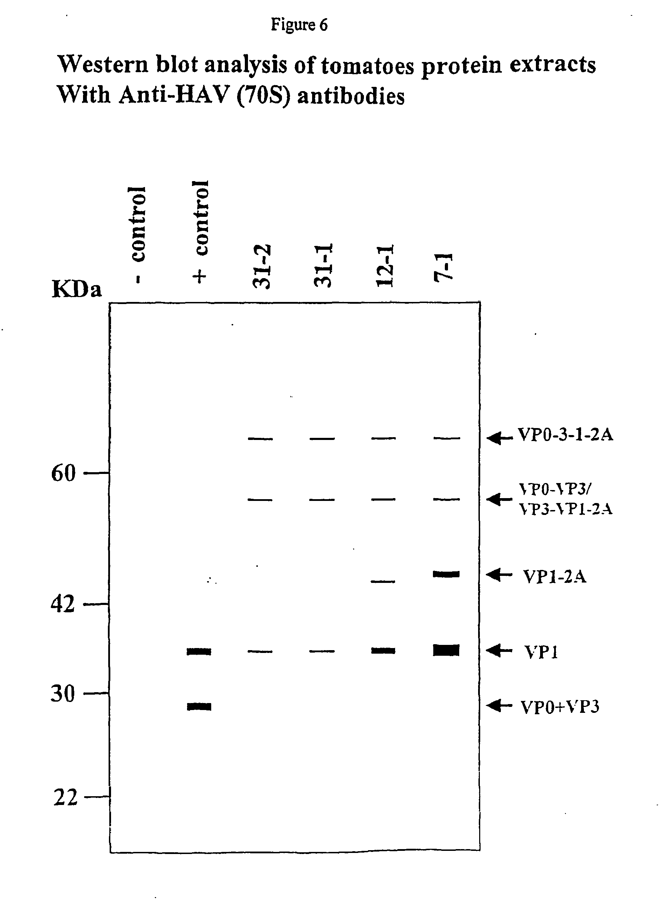

FIG. 6 shows expression of HAV proteins from four different tomato lines, which had previously showed HAV RNA expression. Protein extracts from tomatoes 7-1, 12-1, 31-1 and 31-2 were subjected to western blot analysis, as shown in Figure 6. Tomato tissue taken from all four lines seems to express HAV proteins as detected with the anti-HAV antibody (70S).

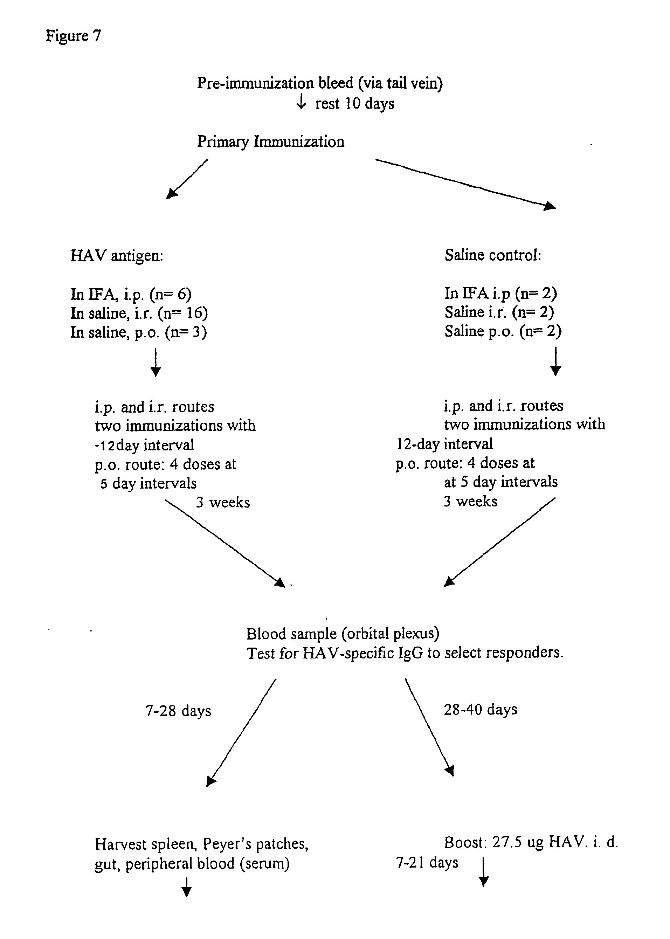

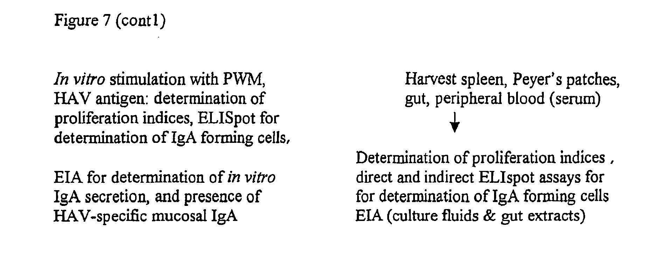

FIG. 7. is a flowchart showing the immunization protocol used in a second set of experiments in which the IgA response of mice to HAV or transgenic tomato HAV vaccine was evaluated.

### DESCRIPTION OF THE PREFERRED EMBODIMENTS

The present invention is of a vaccine produced in edible plant and/or animal products, as well as a method of second generation vaccine development through the production of at least one complete structure of a pathogen in a transgenic plant or animal, as well as by providing the vaccines themselves. Preferably, the present invention enables the production of virus-like particles in edible food plants, through the co-expression of a plurality of proteins and/or of a plurality of portions of such proteins. A "virus-like" particle is therefore herein defined as a group of co-expressed plurality of viral proteins and/or portions of such proteins. The co-expression of viral structural proteins should enhance the proper presentation of viral related antigens to the human immune system.

As a preferred example of the operation of the present invention, a vaccine was developed for hepatitis A virus (HAV). Previous attempts of vaccination with isolated capsid proteins failed for HAV, because it did not result in the production of neutralizing antibodies, which recognize specific structures on the viral particle and which are created only after the assembly of the capsid. To overcome this problem, preferably the present invention includes the construction of two HAV plasmids carrying a non-infectious HAV genome lacking the 5'UTR, for stable transformation of plants. In one plasmid (pGPPatΩHAV) transcription of HAV is driven from the patatin promoter for expression in tomato fruits, and in the second plasmid (pJDHAV) transcription is under the control of the 35SCaMV promoter and should be expressed in the green parts of transgenic plants. For the assessment of the immune response to the HAV transgenes, the crops of transgenic plants were consumed by experimental animals.

According to preferred embodiments of the present invention, the technology which is developed for vaccines can also optionally and more preferably be applied for gene targeting to specific organs, through the consumption of vegetables containing non-infectious viral particles as gene vehicles.

In order to provide a comparison model system for oral administration of any type of vaccine against a pathogen which does not actually attack any portion of the digestive system (mouth, esophagus, stomach, small intestine, large intestine and rectum), a method for orally administering a vaccine against HAV was developed. Next, transgenic plants were constructed with proteins from the outer capsid of HAV as the model pathogen. Finally, these plants were tested in mice as a model mammalian system for their ability to induce an immunogenic response. Each of these stages is discussed in a separate section below.

### SECTION 1: HAV AS A MODEL PATHOGEN

The transgenic plant HAV vaccine was developed as a comparison model system for oral administration of any type of vaccine against a pathogen which does not actually attack any portion of the digestive system (mouth, esophagus, stomach, small intestine, large intestine and rectum). The developed method of oral administration of a regular HAV vaccine is itself novel and non-obvious, as it is the first example of successful oral administration of such a vaccine, which is normally administered through an i.m. (intra-muscular) injection. Thus, the comparison model system is also an inventive vaccine and method for administration thereof, and is part of the present invention.

The gastrointestinal tract is a major port of entry for a large group of viruses which impose a significant health burden. HAV, a plus strand RNA virus, is one such infectious agent. HAV enters the human body through the gastrointestinal system, and migrates to the liver for tissue specific replication, thereby causing liver damage and clinical hepatitis.

In order to immunize the subject against the HAV disease, the comparison model to the present invention involves the rectal administration of a HAV vaccine, such as the currently available HAV vaccine, HAVRIX^{™} (SmithKline Beecham Biologicals). As shown in the results below, the immune system is recognizes the viral nucleocapsid proteins following the presentation of viral peptides by the MHC type I molecules in M type cells or other antigen presenting cells (APC) in the gut epithelium. Vaccines for polioviruses (another member of the picornavirus family) and other viral agents could be developed using the same principle, namely, employing the natural viral tropism to the epithelial cells covering the gastrointestinal tract.

The preferred vaccination strategy according to the present invention involves the exposure of the intestine to HAV related particles, thereby potentially eliciting both an antibody immune response and possibly a cellular immune response as recently shown ("Cellular immune response to hepatitis A vaccine in healthy individuals with delayed seroconversion"; 10^{th} International Symposium on Viral Hepatitis at Atlanta Georgia 9-13 April 2000 by Pagalieroni TG et al. Abstract 011), and optimally generating HAV neutralizing antibodies.

In order to test the method of the present invention, an animal model was developed for the assessment of anti-HAV antibody production in-vivo following rectal administration of HAVRIX^{™} as a method of intestinal exposure to HAV related particles. The following experimental protocol was used. The HAV vaccine was administered to Balb/c mice intraperitoneally (i.p.), intrarectally (i.r.) or intranasally (i.n.), at days 0 and 21. For each treatment, six Balb/c mice received 20 microliters i.n., 100 microliters i.r. or 100 microliters i.p. of a commercial preparation of the HAV vaccine (HAVRIX™), at a concentration of 720 ELISA units per ml.

The mice were then tested for total specific HAV antibodies using a commercial kit (HAVAB, Abbott Laboratories, Diagnostic Division, Abbott Park IL, USA) 35 days after the first vaccination.

As seen in Figure 1, mice which received the vaccine through the i.p. (positive control) or i.r. routes, developed anti-HAV antibodies, whereas mice which received the vaccine intranasally did not develop specific antibodies. Without wishing to be limited to a single hypothesis, these results suggest that following the rectal administration, the viral capsid proteins were presented to the immune system of the mice. This presentation generated anti-HAV antibodies from the IgG class and possibly from the IgA class (although this was not specifically tested). Oral administration of the polio vaccine is known to elicit the generation of antibodies of both the IgG and IgA classes (see for example "Induction of mucosal immunity by inactivated poliovirus vaccine is dependent on previous mucosal contact with live virus"; Herremans TM, Reimerink JH, Buisman AM, Kimman TG, Koopmans MP. J Immunol. 1999 15;162:5011-8).

In cases in which viruses enter through the gut epithelium, mucosal IgA antibodies play an essential role to neutralize the virus at the portal of entry, as they are produced and secreted to the gut lumen.

Systemic (parenteral) administration of vaccines via intradermal (i.d.) or i.m. routes usually gives rise to only IgG class antibodies as evidenced from results of i.m. administration of killed (Salk) polio vaccines. Current HAV vaccine that is also routinely delivered by i.m. injection is thought to produce only IgG class antibodies. Successful immunization against pathogens such as polio virus and HAV which enter via the gut epithelium clearly requires local production of neutralizing antibodies of the IgA class at the portal of entry. Parenteral immunization via the i.d. or i.m. routes which usually gives rise to only a systemic immune response consisting of IgM followed by IgG class antibodies. In contrast, mucosal immunization via the oral (p.o.), or intrarectal (i.r.), or intranasal (i.n.) routes induces both local (mucosal) and systemic immune responses consisting of both IgG and IgA antibodies. In the case of pathogens entering via mucosal (respiratory, gut, genitourinary) routes the latter type of immune response is clearly more beneficial to the host.

The method of the present invention, in which the HAV vaccine is administered to the body through the rectal mucosa, could have a major advantage over the current vaccination program, by blocking the viral portal of entry. In addition, the method of the present invention is able to induce the generation of a successful immune response against HAV, without requiring needles or other invasive methods.

Thus, the experimental results which are described in greater detail below clearly show that the intrarectal route is a suitable method for the generation of protective vaccination against HAV, and also other viruses that enter the body through the gastrointestinal route.

The present invention thus enables the rectal administration of an HAV vaccine such as HAVRIX^{™} for the generation of an anti-HAV immune response, thereby eliciting neutralizing antibodies against HAV.

Without wishing to limit the present invention, a suitable dosage of the HAV vaccine is preferably in the range of from about 0.75 to about 7500 EL.U. of the antigen for each administration, more preferably approximately 75 EL.U. of the antigen, applied to the rectum in a suppository, cream, liquid, tablet, or any other solid, semi-solid or liquid dosage form which is suitable for the administration of HAV antigen to the rectal mucosa.

The method of the present invention is also optionally and preferably suitable for the administration of at least one viral encapsidated gene through the gastrointestinal mucosa of the subject, and particularly through the rectal mucosa. In this optional but preferred method, the viral encapsidated gene or genes is administered to the gastrointestinal mucosa of the subject, in a substantially similar manner as for the previously described HAV vaccine. Thus, the present invention also provides a method for administering one or more viral encapsidated gene or genes to the subject through the gastrointestinal, and particularly the rectal, mucosa.

### SECTION 2: CONSTRUCTION OF A TRANSGENIC PLANT

Transgenic plants were constructed, bearing at least one HAV structure. According to this example, transgenic tomato plants were constructed, for producing HAV viral particles that are non-infectious because their genome excludes about 730nt of its 5' NTR, without which they can neither replicate nor express their own proteins. Elimination of this part of the viral genome does not affect the external structure of the virus. Thus, when these transgenic tomatoes are eaten, the non-infectious viral particles with their nucleocapsid proteins would be ingested as well. The presence of the viral proteins in the gastrointestinal tract should then act as an effective immunogen to induce production of HAV-specific antibodies, as demonstrated in Section 3 below.

### Construction of plasmid vectors with the HAV genome:

A 6.7 kb XbaI-PmeI HAV/7 cDNA fragment lacking the viral 5'UTR, and lacking additional 10 bp from the HAV translational start site, was isolated from plasmid pHAV/7. This fragment was inserted into the modified BSKS plasmid that contained the 10 5' coding nucleotides of Hay/7. Further engineering inserted the 35SCaMV promoter and the Omega (TMV) translation-enhancer box in front of the HAV/7 coding region and a *nos* terminator behind this region. The promoter-HAV terminator combination was finally cloned into a binary vector (for *Agobacterium* mediated genetic-transformation) pGPTV-kan, termed pG35SMIAV. The latter vector was introduced into *Agobacteria* with a helper plasmid and BY-2 tobacco cells were transformed with pG35SnHAV. Kan^{R} cell lines with were selected and established. The presence of HAV sequences in the transfected cells was determined by PCR using primers from the VP1-VP3 viral structural region of the virus (Figure 2).

A similar vector to the pG35SHAV was constructed but with the patatin promoter instead of the 35.S CaUV promoter. This vector was termed pGPPATHAV, and was used to transform tomato plants by the *Agrobacterium* transformation procedure in order to direct the expression of the HAV capsid coding region to tomato fruits. After genetic transformation of tomato tissue with this HAV construct (pGPPATHAV), 17 lines of transgenic tomato plants were derived (4-6 plants in each line). These lines were assessed for the presence of HAV sequences in their genomic DNA Figure 3 depicts a dot blot and hybridization result with radiolabeled HAV cDNA probe of DNA produced from 12 transgenic tomato plant leaves. As seen in the figure all the 12 plants contained HAV DNA sequences. Some plants were als assessed by PCR for HAV-specific sequences (Figure 4).

Concomitantly with the DNA extracted from all the 72 transgenic plants, for the assessment of HAV integrated sequences, the presence of HAV RNA transcripts in the fruits of the positive plants was also detected. Total RNA extracted from tomato plants was probed for HAV-specific RNA sequences by dot blot and hybridization assays (see Figure 5A). To eliminate the possibility that the positive signal seen was due to residual DNA in the RNA samples, the RNA extracted from the tomato fruits was treated with DNase I and the dot blot analysis was repeated (Figure 5B). The samples on A and B were treated with DNase I while the samples on C and D did not. As seen in the figure in some of the RNA samples, the specific signal is weaker after DNase I treatment was performed (e.g A1, A2, A5). However with other samples (A3, A5, A6, A8 and B1), the signal is higher than that of the control tomato (B2).

Plants testing positive for HAV mRNA transcripts were further examined for expression of the inserted RNA. The predicted HAV protein products were detected by Western blot analysis with specific anti-HAV antibodies which included a mouse monoclonal anti-HAV (Biodesign International, Saco, Maine, cat. No. C65885M) and a pooled antiserum produced from Balb/c mice immunized twice at 10-day intervals with 72 EL. U. of HAVRIX^{™} vaccine given i.p.

Figure 6 shows expression of HAV proteins from four different tomato lines, which had previously showed HAV RNA expression. Protein extracts from tomatoes 7-1, 12-1, 31-1 and 31-2 were subjected to western blot analysis, as shown.

The expression of HAV related protein was assessed in these four tomato transgenic lines. Tomato tissue taken from all four lines appears to express HAV proteins as detected with the anti-HAV antibody (70S). Furthermore, the presence of HAV related structural proteins which compose the HAV capsid were detected by this specific antibody, as shown in Figure 6. Thus, the transgenic tomato plants not only express HAV proteins, but more specifically, express those HAV capsid proteins against which an immunogenic response may be expected to be induced.

### SECTION 3: FURTHER EVALUATION OF MUCOSAL IMMUNIZATION STRATEGIES AND PRELIMINARY TESTING OF PLANT-BASED VACCINES IN MICE

As the alum-adsorbed HAVRIX^{™} vaccine for HAV is licensed for delivery only via the i.m. route, and the plant-based vaccine consists of virus-like particles only, experiments were designed to evaluate immune responses to HAV in the gut after presentation as the viral particle without adjuvant (i.e., in a comparable format to the plant-based vaccine). This section describes various tests and analyses that were performed to evaluate mucosal immunization strategies in which HAV virus, alone, was delivered via the i.r. or p.o. route. These experiments were designed to develop evaluation methods for mucosal immunity, and included ELISpot and ELISA tests for measuring, respectively, IgA-forming cells and IgA secretion in the gut mucosa, as well as adapting routine proliferation assays to measure the cellular immune response in both spleen- and gut-derived lymphocytes. This section also describes preliminary testing of plant-based HAV vaccine delivered via the oral route. Experimental methods and results are described in detail below.

### IMMUNIZATION OF MICE

*Antigens:* Female Balb/c mice, aged 4-6 weeks at the outset of experiments were used. Antigens employed were: semi-purified formalin-inactivated HAV (strain HM-175 grown in FRhK4 cells, as in HAVRIXTM vaccine) obtained from a commercial source (Microbix Biosystems, Inc., Toronto, Canada, HAV Grade 2 antigen, cat. No. EL 25-02) which contained 2.2 mg/mL of protein and approx. 6.0 EL.U./mL of HAV antigen according to information provided by the manufacturer. This antigen was used to immunize mice in two different presentation formats. For routine i.p. immunization, the antigen was emulsified 1:1 with incomplete Freund's adjuvant (IFA). For immunization via the i.r. or p.o. routes, the antigen was diluted 1:1 with sterile saline and delivered with a No. 1 surgical catheter attached to a tuberculin syringe. Oral antigen was offered passively and invasive gavage was not employed. For all immunizations in this series of experiments dosage volume was 100 microL (containing approx. 110 micrograms of total protein or 0.3 EL. U. of HAV antigen). Transgenic plant HAV vaccine produced by two tomato strains (2-21 and 3-12), both of which were shown to express mRNA for the HAV insert, were also evaluated. For immunization, approx. 0.4 g of lyophilized transgenic plant material was suspended in 4 mL of sterile saline and sonicated on ice for 3 min using 10-second bursts. The resultant material was centrifuged and the supernatant used to immunize mice, giving each animal 100 microliters p.o. Sterile saline either emulsified with IFA (for i.p. injections) or given alone (for i.r. or p.o. routes) served as a negative control antigen.

*Immunization protocol and schedules:* The immunization protocol, schedule and numbers of animals used are as follows. Animals immunized via the i.p. or i.r. routes received two doses at 12-day intervals while animals immunized orally received 4 doses at 5-day intervals. Three weeks after the final immunizations approx. 0.5 mL of blood was obtained from the tail vein of each mouse. To determine which mice were likely to have responded to immunization sera were prepared and tested using a commercial competitive inhibition enzyme immunoassay (ETI-AB-HAVK-3 [anti-HAV], Sorin BioMedica, Italy) designed for measuring HAV-specific IgG in human serum with a sensitivity of <20 WHO mIU/mL. As the assay is a competitive inhibition method, in which the antibody to be tested competitively inhibits binding of one of the kit components, the assay was used to determine if any of the mice had produced HAV-specific IgG capable of inhibiting the binding of the assay reagent.

While none of the animals tested was definitively positive for HAV-specific IgG by this method, two of the mice immunized with HAV/IFA via the i.p. route had borderline levels of specific IgG. As these negative results did not preclude the possibility that the mice may have responded in the mucosal immune compartment, the animals were subsequently randomized among the treatments and tested in two groups. One group was killed in subgroups of 6-8 mice over a period spanning 7-28 days after the last immunization. Spleens and Peyer's patches were harvested and cell populations were prepared for stimulation *in vitro* with HAV or control antigens (see below) and evaluation of the HAV-specific IgA response using ELISpot and ELISA, and HAV-specific proliferation indices as described in detail below. The second group of mice was allowed to rest a further three weeks, then received a single i.d. boost of HAV antigen (27.5 ug of total protein or 0.6 EL. U.). These mice were killed as groups of 6-8 over a period of 7-21 days. This latter group was designated as "*in vivo* boosted" in contrast to the former group whose cells were restimulated with HAV antigen *in vitro.*

### PREPARATION OF SPLEEN CELL AND PEYER'S PATCH CELL POPULATIONS

Animals were killed by an overdose of chloral hydrate. Blood was collected by cardiac puncture, allowed to clot overnight at 4 C. Serum was prepared and kept frozen at -20° C until tested. The spleen and Peyer's patches (approx. 4-8 per animal) were removed into sterile RPMI-1640 medium (GIBCO/BRL, Biological Industries, Beit Haemek, Israel) containing antibiotics (penicillin, 100 U/mL and streptomycin, 100 micrograms per mL, GIBCO/BRL) and transported to the laboratory for further processing. The organs were washed once in sterile RPMI. Spleen cell (SPLC) suspensions were prepared by teasing apart the spleen in 5 mL of RPMI + antibiotics using sterile forceps. The suspension was transferred into a sterile conical bottomed 15 mL tube and the tissue debris allowed to settle. The supernatant containing suspended SPLC was transferred into a fresh tube and the cells were pelleted by centrifugation (1500 rpm, 7 min) at room temperature. The cells were washed twice by centrifugation (1500 rpm, 5 min) through RPMI + antibiotics then viable cell numbers were determined by Trypan Blue exclusion using a Neubauer hemocytometer. The SPLC were then resuspended in 10 mL of the same medium containing 10% fetal calf serum (FCS, HyClone Laboratories, Tarom Applied Technologies, Petah Tikvah, Israel) at a concentration of 1 x 10⁶ cells/mL for use in subsequent assays.

Similarly, Peyer's patches were teased apart in 5 mL of RPMI + antibiotics containing 1.5 mg/mL of Dispase (Sigma Chemical Co., Israel). The Peyer's patch cell (PPC) suspension was transferred into a conical-bottomed 15 mL tube and incubated with shaking for 45-60 min at 37° C to dissociate cells from the fibrous structure of the tissue. The PPC were then pelleted by centrifugation and washed twice through RPMI + antibiotics and numbers of viable cells were determined as described above. As PPC numbers were usually limited, the final suspensions in 4.5 mL of RPMI containing antibiotics and 10% FCS, which ranged in numbers from 1 x 10⁴ to 5 x 10⁵ cells/mL, were used in assays without further adjustment of numbers.

### IN VITRO STIMULATION OF SPLC AND PPC IN BULK CULTURE

After determination of initial numbers of cells per milliliter, SPLC and PPC preparations from each mouse were pipetted in 1-mL aliquots into 5 wells of flat-bottomed sterile 24-well tissue culture plates. For each cell population, separate wells received: medium only (unstimulated negative control); pokeweed mitogen (Sigma) at a final concentration of 10 micrograms per mL; and HAV antigen (Microbix) diluted to give final concentrations per well of 11, 5.5, 2.8, and 1.4 micrograms per mL. The plates were incubated for 5 days at 37 C, 5% CO₂ in air. At the end of this culture period, 0.7 mL of supernatant was removed from each well and stored at -20° C for determination of HAV-specific IgA by ELISA (see below). For assays in which HAV-specific IgA forming cells were evaluated after *in vitro* stimulation the cells were treated as follows. Cells from each well were harvested into conical-bottomed tubes, washed once by centrifugation through RPMI + antibiotics and resuspended in 1 mL of RPMI containing antibiotics and 10% PCS. Viable cell numbers after *in vitro* stimulation were determined by Trypan Blue exclusion and related to cell densities in the initial cell population to give a stimulation index (SI). SI's ≥ 2.0 were considered a positive response to stimulation. The cell populations were adjusted (whenever cell numbers permitted) to 1 x 10⁶ cells/mL and used in ELISpot assays for determination of presence and numbers of HAV-specific IgA forming cells as described below.

### PROLIFERATION ASSAYS

Proliferation assays were performed as follows for experiments where the response to *in vivo* boosting with HAV was evaluated. SPLC and PPC (when in sufficient quantity) were adjusted to 1 x 10⁶ cells/mL in RPMI containing antibiotics and 10% FCS. Proliferation assays were performed in flat-bottomed 96-well tissue culture plates. Each well received 100 microliters of cell suspension, 100 microliters of medium (as above), and 10 microliters of one of the following: medium only (unstimulated control); phytohemagglutinin (Sigma) prepared to give a final well concentration of 10 micrograms per mL; or HAV antigen (Microbix) diluted to give final well concentrations of 11, 5.5, 2.8, and 1.4 micrograms per mL. All concentrations are expressed as micrograms of protein/mL. Duplicate wells were set up for each stimulant. The plates were incubated for 5 days at 37° C in 5% CO₂ in air. To determine the extent of cellular proliferation, 2 micro-Ci of ³H thymidine (Amersham, Israel) was added to each well during the last 18-24 hours of culture. The cells were harvested by hypotonic lysis and their DNA collected onto glass fiber filters for determination of incorporated radioactivity by liquid scintillation counting. Stimulation indices (SI) were determined by dividing the averaged (over duplicates) cpm obtained for the stimulated wells by the averaged cpm obtained for the negative control (medium only) wells. SI's ≥ 2.0 were considered positive.

### ELISpot ASSAYS

To detect cells producing specific IgA antibodies in both the spleen and Peyer's patches, an ELISpot assay was adapted from a standard protocol (Lewis DJM & Hayward CMM, Stimulation of Mucosal Immunity. In: Vaccine Protocols; A. Robinson, GH Farrar, CN Wiblin, Eds., Humana Press, Totowa, NJ, 1996, pp 187-195). The principle of this assay is that cells forming specific IgA will secrete it onto the surface of antigen-coated microplate wells, leaving an "imprint" which may be detected by conventional enzyme-linked immunosorbent (ELISA) techniques. Wells of 96-well polystyrene tissue culture plates (Costar 3596, Corning Inc., Corning, NY) were coated overnight with HAV antigen (Microbix), or FRhK4 lysate, (prepared by freeze thawing the cells and clarifying the supernatant by centrifugation at 15,000 rpm for 20 min), diluted to 20 micrograms per mL of protein in standard ELISA coating buffer (HCO₂⁻/CO₃²⁻, pH 9.6). Negative control wells received coating buffer only. Coating volume was 100 microliters per well and duplicate wells were coated for each antigen or control. The following day the antigens were discarded and the wells were washed 4 times with sterile phosphate buffered saline (PBS, pH 7.4). The wells were subsequently blocked for 2-4 h at room temperature with 5% FCS in sterile PBS (250 microliters per well). The blocking solution was discarded and SPLC or PPC suspensions were added to the wells and the plates were incubated for 24 h at 37° C in 5% CO₂ in air, taking care to keep the plates level and undisturbed during this period. SPLC were incubated at a density of 100,000 cells/well in a volume of 100 microliters per well while PPC were used at densities of 1000-50,000 cells/well (100 microliters per well) according to their yields. At the end of the incubation period the cells were discarded and the microplate wells were washed 5 times in PBS containing 0.05% Tween 20 (PBST).

To detect spots where antigen-specific IgA-forming cells had secreted their antibodies into the antigen-coated microplate wells, goat-anti-mouse IgA (affinity purified from Kirkegaard & Perry Labs [KPL]., Gaithersburg, MD) diluted 1:500 in PBST containing 5% normal rabbit serum (NRS, Biological Industries, Beit Haemek, Israel), 100 microliters per well was added and the microplates were incubated overnight at 4° C. The antibody was discarded and the wells were washed 5 times with PBST. Next, alkaline phosphatase-conjugated rabbit anti-goat IgG (affinity-purified, KPL) diluted to 1:1000 in PBST containing 5% NRS, 100 microliters per well, was added and the microplates were incubated for 2 h at room temperature. The antibody solution was discarded and the microplates were washed 5 times with PBST. To detect ELIspots, alkaline phosphatase substrate (BCIP/NBT SigmaFast, Sigma) prepared according to the manufacturer's directions, was.added (100 microliters per well), and the enzymatic reaction was allowed to proceed at room temperature in the dark for 45 min before stopping it by rinsing the plates 3 times in distilled water. The plates were covered with aluminum foil and stored at 4° C until ELIspot counting. ELIspots were enumerated using an inverted microscope with 400x magnification. As nonspecific background reactivity in ELIspot assays is inherent, all assays contained controls for reactivity with FRhK4 antigens (present in the original immunogen) and nonspecific binding to other assay constituents (coating buffer control). ELIspots in duplicate wells for HAV and control antigens were counted and averaged. To calculate the net number of HAV-specific ELIspots, the averaged number of ELIspots in the control wells were averaged then this average was subtracted from the averaged number of ELIspots calculated in wells coated with HAV antigen. The net HAV-specific ELIspot number was reported (data tables).

### ELISA

During the course of these experiments, preliminary development of an enzyme-linked immunosorbent assay (ELISA) for detecting HAV-specific IgA antibodies in mouse serum or in tissue culture supernatants was undertaken. Wells of polystyrene ELISA microplates (Nunc Immunopolysorp, Fisher Scientific, Israel) were coated overnight at 4° C with HAV antigen (Microbix) or FRhK4 antigen, diluted to 20 micrograms per mL of protein in carbonate/bicarbonate coating buffer as described above (100 microliters per well, in duplicates). Negative control wells received coating buffer only. The antigen or control solutions were discarded and the wells were washed three times in PBS containing 0.05% Tween 20 (PBST). Wells were subsequently blocked for 1 h at room temperature with 250 microliters of blocking buffer (5% NRS in PBST). The blocking solution was discarded and the wells were washed 5 times with PBST. Tissue culture fluids to be tested for *in vitro* production of HAV-specific IgA or dilutions of mouse serum from immunized animals (1:20 and 1:200 in blocking buffer) were subsequently added (100 microliters per well, single determinations for each antigen and negative control) and the plates were incubated overnight at 4° C. These solutions were discarded and the wells washed 5 times in PBST as before.

Next, goat anti-mouse IgA (KPL) diluted to 1:500 in blocking buffer (100 microliters per well) was added and the plates were incubated for 2 h at room temperature. The wells were emptied and washed 5 times with PBST. Alkaline phosphatase (AP)-conjugated goat anti-rabbit IgG (KPL, 1:1000 dilution in blocking buffer, 100 microliters per well) was added and the plates were incubated for a further 2 h at room temperature. After discarding the AP conjugate and washing the wells 5 times with PBST, AP substrate (SigmaFast NBT substrate, Sigma, prepared according to the manufacturer's directions was added and the plate was incubated for 1 h at room temperature. Absorbance at 405 nm (A₄₀₅) determined using an ELISA microplate reader (Tecan Spectra Rainbow). For each animal and treatment results were expressed as a signal-to-noise (S/N) ratio obtained by dividing the A₄₀₅ determined with antigen (either HAV or FRhK4) by the A₄₀₅ recorded in wells receiving coating buffer only. S/N ratios ≥ 2.0 were considered positive.

### Results

### Results of Bulk Culture Experiments: "Positive Controls" (mice immunized with HAV through the i.p. route).

| Immunization | IgA Stimulation Index (SI)^{b} | | Specific IGA (S/N)^{c} | | No. of ELIspots (Mean^{d}) | |
|---|---|---|---|---|---|---|
| Stimulant^{a} | SPLC | PPC | SPLC | PPC | SPLC | PPC |
| HAV/IFA i.p. #1: | | | | | | |
| None | - | - | 1.7 | 2.1 | 0 | 0 |
| Mitogen (10 ug/ml)^{e} | 0.5 | 1.9 | 1.7 | 1.8 | 1.5 | 0 |
| HAV (11 ug/mL) | 0.5 | 2.3 | 2.3 | 2.0 | 10.1 | 1.5 |
| HAV (5.5 ug/mL) | 0.5 | 0.8 | 1.7 | 1.5 | 17 | 0 |
| HAV (2.8 ug/mL) | 0.7 | 2.6 | 1.8 | 1.6 | 0 | 0 |
| HAV (1.4 ug/mL) | ND | ND | ND | ND | ND | ND |
| | | | | | | |
| HAV/IFA i. p. #2: | | | | | | |
| None | - | - | 1.5 | 1.4 | 5 | 0 |
| Mitogen (10 ug/ml)^{e} | 1.3 | 1.6 | 1.5 | 1.0 | 0 | 0 |
| HAV (11 ug/mL) | 2.3 | 1.3 | 1.6 | 1.6 | 25.5 | 0.5 |
| HAV (5.5 ug/mL) | 1.5 | 2.2 | 1.5 | 1.3 | 11.2 | 0 |
| HAV (2.8 ug/mL) | 1.0 | 1.9 | 2.0 | 1.6 | 21.7 | 0 |
| HAV (1.4 ug/mL) | ND | ND | ND | ND | ND | ND |

| | | | | | | |
|---|---|---|---|---|---|---|
| ^{a}Immunization protocol (for animal) and stimulant used (final concentration) in bulk culture for 5-6 days before testing. ^{b}Stimulation index (SI) determined from no. of viable cells/mL (as determined by Trypan Blue exclusion hemocytometer counts) in stimulated wells divided by the no. of viable cells/mL in unstimulated (medium only) wells. SI≥ 2.0 considered positive. ^{c}HAV-specific IgA S/N (signal:noise ratio) as determined using EIA by dividing A405 nm measured in wells coated with HAV antigen by A405nm measured in wells receiving coating buffer only. S/N ≥ 2.0 considered positive. ^{d}No. of positive ELISpots: average no. of ELISpots counted in wells coated with HAV above averaged background of ELISpots counted in wells coated with FRhK4 antigen or coating buffer only. ^{e}In bulk culture experiments mitogen was pokeweed mitogen (PWM); in proliferation assays involving mice boosted *in vivo* by an additional i. d. injection of HAV, the mitogen was phytohemagglutinin (PHA). SPLC= spleen cells PPC= Peyer's patch cells | | | | | | |

### Results of Bulk Culture Experiments: Mucosally-immunized mice.

| Immunization | IgA Stimulation Index (SI)^{b} | | Specific IGA (S/N)^{c} | | No. of ELIspots (Mean)^{d} | |
|---|---|---|---|---|---|---|
| Stimulant^{a} | | | | | | |
| | SPLC | PPC | SPLC | PPC | SPLC | PPC |
| HAVi. r. #1: | | | | | | |
| None | - | - | 1.4 | 1.1 | 0 | 0 |
| Mitogen (10 ug/ml)° | 1.1 | 0.3 | 1.3 | 2.3 | 0 | 0 |
| HAV (11 ug/mL) | 1.2 | 0.6 | 1.6 | 1.8 | 43 | 0 |
| HAV (5.5 ug/mL) | 1.0 | 1.2 | 1.4 | 1.7 | 31.3 | 0 |
| HAV (2.8 ug/mL) | 2.2 | 1.3 | 1.4 | 1.5 | 16.5 | 0 |
| HAV (1.4 ug/mL) | ND | ND | ND | ND | ND | ND |
| | | | | | | |
| HAV i. r. #2: | | | | | | |
| None | - | - | 1.3 | 1.5 | 65.7 | 0 |
| Mitogen (10 ug/ml)^{e} | 0.1 | 3.0 | 1.1 | 1.6 | 0 | 0 |
| HAV (11 ug/mL) | 1.3 | 8.0 | 1.4 | 1.5 | 20.2 | 0 |
| HAV (5.5 ug/mL) | 1.0 | 5.4 | 1.5 | 1.7 | 39 | 0 |
| HAV (2.8 ug/mL) | 1.0 | 6.1 | 1.4 | 2.2 | 55.2 | 0 |
| HAV (1.4 ug/mL) | ND | ND | ND | ND | ND | ND |
| | | | | | | |
| HAV i. r. #3: | | | | | | |
| None | - | - | 1.0 | 1.1 | 0 | 0 |
| Mitogen (10 ug/ml)^{e} | 0.1 | 2.0 | 0.9 | 1.9 | 0 | 0 |
| HAV (11 ug/mL) | 1.1 | 5.0 | 0.9 | 1.3 | 0 | 0 |
| HAV (5.5 ug/mL) | 1.2 | 3.0 , | 2.0 | 1.6 | 0 | 0 |
| HAV (2.8 ug/mL) | 0.4 | 3.9 | 1.1 | 1.7 | 17 | 0 |
| HAV (1.4 ug/mL) | ND | ND | ND | ND | ND | ND |
| | | | | | | |
| HAV i.r. #4: | | | | | | |
| None | - | - | 1.0 | 1.0 | 1.5 | 0 |
| Mitogen (10 ug/ml)^{e} | 1.1 | 1.7 | 1.3 | 1.4 | 0 | 0 |
| HAV (11 ug/mL) | ND | ND | ND | ND | ND | ND |
| HAV (5.5 ug/mL) | 1.2 | 2.4 | 1.4 | 1.4 | 2 | 0 |
| HAV (2.8 ug/mL) | ND | ND | ND | ND | ND | ND |
| HAV (1.4 ug/mL) | ND | ND | ND | ND | ND | ND |
| | | | | | | |
| HAV i. r. #5: | | | | | | |
| None | - | - | 1.4 | 1.5 | 0.5 | 0 |
| Mitogen (10 ug/ml)^{e} | 0.8 | 1.4 | 1.3 | 1.4 | 0 | 0 |
| HAV (11 ug/mL) | ND | ND | ND | ND | ND | ND |
| HAV (5.5 ug/mL) | 1.1 | 1.6 | 1.4 | 1.4 | 0 | 0 |
| HAV(2.8ug/mL) | ND | ND | ND | ND | ND | ND |
| HAV (1.4 ug/mL) | ND | ND | ND | ND | ND | ND |
| | | | | | | |
| HAV p.o: | | | | | | |
| None | - | - | 1.4 | 1.5 | 24 | 0 |
| Mitogen (10 ug/ml)^{e} | 0.4 | 4.2 | 1.6 | 0.9 | 0 | 0 |
| HAV (11 ug/mL) | ND | ND | ND | ND | ND | ND |
| HAV (5.5 ug/mL) | 1.2 | 1.7 | 1.5 | 1.4 | 17 | 0 |
| HAV (2.8 ug/mL) | ND | ND | ND | ND | ND | ND |
| HAV (1.4 ug/mL) | ND | ND | ND | ND | ND | ND |
| | | | | | | |

| | | | | | | |
|---|---|---|---|---|---|---|
| ^{a}Immunization protocol (for animal) and stimulant used (final concentration) in bulk culture for 5-6 days before testing. ^{b}Stimulation index (SI) determined from no. of viable cells/mL (as determined by Trypan Blue exclusion hemocytometer counts) in stimulated wells divided by the no. of viable cells/mL in unstimulated (medium only) wells. SI≥ 2.0 considered positive. ^{c}HAV-specific IgA S/N (signal:noise ratio) as determined using EIA by dividing A405 nm measured in wells coated with HAV antigen by A405nm measured in wells receiving coating buffer only. S/N ≥ 2.0 considered positive. ^{d}No. of positive ELISpots: average no. of ELISpots counted in wells coated with HAV above averaged background of ELISpots counted in wells coated with FRbK4 antigen or coating buffer only. ^{e}In bulk culture experiments mitogen was pokeweed mitogen (PWM); in proliferation assays involving mice boosted *in vivo* by an additional L d. injection of HAV, the mitogen was phytohemagglutinin (PHA). | | | | | | |

### Results of Bulk Culture Experiments: Negative Controls.

| Immunization | IgA Stimulation Index (SI)^{b} | | Specific IGA (S/N)^{c} | | No. of ELIspots (Mean^{d}) | |
|---|---|---|---|---|---|---|
| Stimulant^{a} | SPLC | PPC | SPLC | PPC | SPLC | PPC |
| Saline/IFA i.p.: | | | | | | |
| None | - | - | 1.8 | 1.5 | 0 | 0 |
| Mitogen (10 ug/ml)^{e} | 0.3 | 1.0 | 1.7 | 1.5 | 0 | 0 |
| HAV (11 ug/mL) | 1.6 | 1.1 | 1.7 | 1.4 | 4.2 | 0 |
| HAV (5.5 ug/mL) | 0.6 | 2.0 | 1.6 | 1.4 | 1.8 | 0 |
| HAV (2.8 ug/mL) | 0.8 | 1.2 | 1.5 | 1.4 | 0 | 0 |
| HAV (1.4 ug/mL) | ND | ND | ND | ND | ND | ND |
| | | | | | | |
| Saline p.o.: | | | | | | |
| None | - | - | 1.4 | 1.5 | 0 | 0 |
| Mitogen (10 ug/ml)^{e} | 1.0 | 2.3 | 1.6 | 0.9 | 0 | 0 |
| HAV (11 ug/mL) | ND | ND | ND | ND | ND | ND |
| HAV (5.5 ug/mL) | 0.9 | 1.3 | 1.5 | 1.4 | 0 | 0 |
| HAV (2.8 ug/mL) | ND | ND | ND | ND | ND | ND |
| HAV (1.4 ug/mL) | ND | ND | ND | ND | ND | ND |

| | | | | | | |
|---|---|---|---|---|---|---|
| ^{a}Immunization protocol (for animal) and stimulant used (final concentration) in bulk culture for 5-6 days before testing. ^{b}Stimulation index (SI) determined from no. of viable cells/mL (as determined by Trypan Blue exclusion hemocytometer counts) in stimulated wells divided by the no. of viable cells/mL in unstimulated (medium only) wells. SI≥ 2.0 considered positive. ^{c}HAV-specific IgA S/N (signal:noise ratio) as determined using EIA by dividing A 405 nm measured in wells coated with HAV antigen by A405nm measured in wells receiving coating buffer only. S/N ≥ 2.0 considered positive. ^{d}No. of positive ELISpots: average no. of ELISpots counted in wells coated with HAV above averaged background of ELISpots counted in wells coated with FRhK4 antigen or coating buffer only. ^{e}In bulk culture experiments mitogen was pokeweed mitogen (PWM); in proliferation assays involving mice boosted *in vivo* by an additional i. d. injection of HAV, the mitogen was phytohemagglutinin (PHA). | | | | | | |

### Results of Experiments Following i.d. Boosting of Immunity to HAV: "Positive Controls".

| Immunization | IgA Stimulation Index (SI)^{b} | | Specific IGA (S/N)^{c} | | No. of ELIspots (Mean^{d}) | |
|---|---|---|---|---|---|---|
| Stimulant^{a} | SPLC | PPC | SPLC | PPC | SPLC | PPC |
| HAV/IFA i.p. #1: | | | | | | |
| None | - | - | 4.4 | ND | 12.3 | 2.5 |
| Mitogen (10 ug/ml) | 2.6 | 1.4 | 3.0 | ND | ND | ND |
| HAV (11 ug/mL) | 2.0 | 0.8 | 2.5 | ND | ND | ND |
| HAV (5.5 ug/mL) | 0.7 | 1.2 | 2.5 | ND | ND | ND |
| HAV (2.8 ug/mL) | 0.9 | 1.1 | 3.5 | ND | ND | ND |
| HAV (1.4 ug/mL) | 1.2 | 1.8 | 2.2 | ND | ND | ND |
| | | | | | | |
| HAV/IFA i.p. #2.: | | | | | | |
| None | - | - | 3.2 | ND | 0 | 7.5 |
| Mitogen (10 ug/ml)^{e} | 1.3 | 2.8 | 2.2 | ND | ND | ND |
| HAV (11 ug/mL) | 0.9 | 2.4 | 1.7 | ND | ND | ND |
| HAV (5.5 ug/mL) | 1.4 | 3.0 | 1.7 | ND | ND | ND |
| HAV(2.8 ug/mL) | 1.1 | 2.7 | 1.6 | ND | ND | ND |
| HAV (1.4 ug/mL) | 1.2 | 4.2 | 1.6 | ND | ND | ND |

| | | | | | | |
|---|---|---|---|---|---|---|
| ^{a}Immunization protocol by animal. Animals were boosted (i.d.) with 27.5 ug of HAV protein (Microbix HAV antigen) 10-28 days before testing. ^{b}Stimulation index (SI) determined from ³H-TdR incorporation experiments: no. of cpm incorporated into cells in stimulated wells divided by the no. of cpm incorporated into cells in unstimulated (medium only) wells. Mitogen used was PHA. SI≥ 2.0 considered positive. ^{c}HAV-specific IgA S/N (signal:noise ratio) as determined using EIA by dividing A405 nm measured in wells coated with HAV antigen by A405nm measured in wells receiving coating buffer only. Mitogen used was PWM. S/N ≥ 2.0 considered positive. ^{d}No. of positive ELISpots: average no. of ELISpots counted in wells coated with HAV above averaged background of ELISpots counted in wells coated with FRhK4 antigen or coating buffer only. | | | | | | |

### Results of Experiments Following i.d. Boosting of Immunity to HAV: Mucosally-Immunized Mice.

| Immunization | IgA Stimulation Index (SI)^{b} | | Specific IGA (S/N)^{c} | | No. of ELIspots (Mean^{d}) | |
|---|---|---|---|---|---|---|
| Stimulant^{a} | SPLC | PPC | SPLC | PPC | SPLC | PPC |
| HAV i.r. #1: | | | | | | |
| None | - | - | 2.0 | ND | 2.0 | 1.5 |
| Mitogen (10 ug/ml) | 0.9 | 4.5 | 1.7 | ND | ND | ND |
| HAV (11 ug/mL) | 0.6 | 2.1 | 1.1 | ND | ND | ND |
| HAV (5.5 ug/mL) | 0.8 | 3.0 | 1.3 | ND | ND | ND |
| HAV (2.8 ug/mL) | 1.1 | 3.9 | 1.1 | ND | ND | ND |
| HAV (1.4 ug/mL) | 0.6 | 4.1 | 1.2 | ND | ND | ND |
| | | | | | | |
| HAV i. r. #2.: | | | | | | |
| None | - | - | 1.1 | ND | 0 | 0.3 |
| Mitogen (10 ug/ml)^{e} | 1.1 | 4.1 | 1.3 | ND | ND | ND |
| HAV (11 ug/mL) | 1.0 | 0.8 | 1.2 | ND | ND | ND |
| HAV (5.5 ug/mL) | 0.9 | 1.2 | 1.3 | ND | ND | ND |
| HAV (2.8 ug/mL) | 0.7 | 0.6 | 1.0 | ND | ND | ND |
| HAV (1.4 ug/mL) | 0.7 | 1.0 | 1.5 | ND | ND | ND |
| | | | | | | |
| HAV *i.r*. #3.: | | | | | | |
| None | - | - | 1.5 | ND | 4.8 | 0 |
| Mitogen (10 ug/ml)° | 2.1 | 0.5 | 1.5 | ND | ND | ND |
| HAV (11 ug/mL) | 2.0 | 0.5 | 1.5 | ND | ND | ND |
| HAV (5.5 ug/mL) | 2.3 | 0.7 | 1.3 | ND | ND | ND |
| HAV (2.8 ug/mL) | 8.0 | 1.1 | 1.3 | ND | ND | ND |
| HAV (1.4 ug/mL) | 2.2 | 1.3 | 1.3 | ND | ND | ND |
| | | | | | | |
| HAV i. r. #4.: | | | | | | |
| None | - | - | 1.2 | ND | 1.0 | 2 |
| Mitogen (10 ug/ml)° | 1.7 | 2.4 | 1.3 | ND | ND | ND |
| HAV (11 ug/mL) | 2.4 | 1.0 | 1.3 | ND | ND | ND |
| HAV (5.5 ug/mL) | 1.7 | 1.2 | 1.3 | ND | ND | ND |
| HAV (2.8 ug/mL) | 8.0 | 1.1 | 1.3 | ND | ND | ND |
| HAV (1.4 ug/mL) | 1.7 | 1.4 | 1.2 | ND | ND | ND |
| | | | | | | |
| HAV i. r. #5.: | | | | | | |
| None | - | - | 1.0 | ND | 0 | 0 |
| Mitogen (10ug/ml)^{e} | 1.8 | 0.7 | 1.1 | ND | ND | ND |
| HAV(11 ug/mL) | 1.2 | 0.5 | 1.8 | ND | ND | ND |
| HAV (5.5 ug/mL) | 1.1 | 0.8 | 1.5 | ND | ND | ND |
| HAV (2.8 ug/mL) | 1.1 | 0.8 | 1.8 | ND | ND | ND |
| HAV (1.4 ug/mL) | 0.9 | 0.8 | 1.3 | ND | ND | ND |
| | | | | | | |
| HAV i. r. #6: | | | | | | |
| None | - | - | 1.3 | ND | 0 | 0 |
| Mitogen (10 ug/ml) | 0.6 | 0.7 | 1.0 | ND | ND | ND |
| HAV (11 ug/mL) | 0.6 | 0.8 | 1.3 | ND | ND | ND |
| HAV (5.5 ug/mL) | 1.0 | 0.7 | 1.4 | ND | ND | ND |
| HAV (2.8 ug/mL) | 0.7 | 0.9 | 1.4 | ND | ND | ND |
| HAV (1.4 ug/mL) | 0.7 | 0.7 | 1.2 | ND | ND | ND |
| | | | | | | |
| HAV i. r. #7: | | | | | | |
| None | - | - | 2.7 | ND | 5.8 | 0.5 |
| Mitogen (10 ug/ml) | 2.0 | 0.8 | 2.6 | ND | ND | ND |
| HAV (11 ug/mL) | 1.6 | 1.1 | 2.7 | ND | ND | ND |
| HAV (5.5 ug/mL) | 2.3 | 0.9 | 2.9 | ND | ND | ND |
| HAV (2.8 ug/mL) | 1.6 | 0.9 | 2.7 | ND | ND | ND |
| HAV (1.4 ug/mL) | 1.0 | 1.1 | 2.6 | ND | ND | ND |
| | | | | | | |
| HAV i. r. #8: | | | | | | |
| None | - | - | 2.4 | ND | 2.5 | 7.5 |
| Mitogen (10 ug/ml) | 1.2 | 1.8 | 2.8 | ND | ND | ND |
| HAV (11 ug/mL) | 1.2 | 1.9 | 2.4 | ND | ND | ND |
| HAV (5.5 ug/mL) | 1.5 | 1.7 | 2.7 | ND | ND | ND |
| HAV (2.8 ug/mL) | 1.0 | 3.1 | 2.8 | ND | ND | ND |
| HAV (1.4 ug/mL) | 1.3 | 2.8 | 2.7 | ND | ND | ND |
| | | | | | | |
| HAV i. r. #9: | | | | | | |
| None | - | - | 2.8 | ND | 1.3 | 2.0 |
| Mitogen (10 ug/ml) | 0.8 | 3.3 | 2.8 | ND | ND | ND |
| HAV (11 ug/mL) | 0.8 | 3.1 | 2.8 | ND | ND | ND |
| HAV (5.5 ug/mL) | 0.8 | 2.7 | 2.9 | ND | ND | ND |
| HAV (2.8 ug/mL) | 0.8 | 3.1 | 3.6 | ND | ND | ND |
| HAV (1.4 ug/mL) | 0.5 | 2.8 | 3.0 | ND | ND | ND |
| | | | | | | |
| HAV i. r. #10: | | | | | | |
| None | - | - | 2.9 | ND | 0.5 | 4.5 |
| Mitogen (10 ug/ml) | 1.3 | 1.7 | 2.6 | ND | ND | ND |
| HAV (11 ug/mL) | 1.2 | 1.1 | 2.8 | ND | ND | ND |
| HAV (5.5 ug/mL) | 1.4 | 1.8 | 2.5 | ND | ND | ND |
| HAV (2.8 ug/mL) | 1.2 | 1.5 | 2.9 | ND | ND | ND |
| HAV (1.4 ug/mL) | 1.3 | 1.5 | 2.9 | ND | ND | ND |
| | | | | | | |
| HAV p.o.#1: | | | | | | |
| None | - | - | 3.6 | ND | 1.5 | 7.8 |
| Mitogen (10 ug/ml) | 1.2 | 2.9 | 2.9 | ND | ND | ND |
| HAV (11 ug/mL) | 0.9 | 11.6 | 2.6 | ND | ND | ND |
| HAV (5.5 ug/mL) | 1.1 | 1.1 | 2.6 | ND | ND | ND |
| HAV (2.8 ug/mL) | 0.7 | 0.9 | 2.5 | ND | ND | ND |
| HAV (1.4 ug/mL) | 1.2 | 1.4 | 3.1 | ND | ND | ND |
| | | | | | | |
| HAV p. o.#2: | | | | | | |
| None | - | - | 2.8 | ND | 2.3 | 11.8 |
| Mitogen (10 ug/ml) | 1.5 | 4.1 | 3.3 | ND | ND | ND |
| HAV (11 ug/mL) | 1.2 | 1.7 | 2.7 | ND | ND | ND |
| HAV (5.5 ug/mL) | 1.8 | 1.9 | 3.2 | ND | ND | ND |
| HAV (2.8 ug/mL) | 0.9 | 2.0 | 2.7 | ND | ND | ND |
| HAV (1.4 ug/mL) | 1.1 | 2.2 | 3.0 | ND | ND | ND |
| | | | | | | |
| T12-2 p.o #1.: | | | | | | |
| None | - | - | 2.6 | ND | 5.0 | 6.0 |
| Mitogen (10 ug/ml) | 0.1 | 1.6 | 3.2 | ND | ND | ND |
| HAV (11 ug/mL) | 2.4 | 1.5 | 3.2 | ND | ND | ND |
| HAV (5.5 ug/mL) | 2.5 | 1.0 | 3.0 | ND | ND | ND |
| HAV (2.8 ug/mL) | 1.5 | 1.3 | 3.1 | ND | ND | ND |
| HAV (1.4 ug/mL) | 2.0 | 1.9 | 3.1 | ND | ND | ND |
| | | | | | | |
| T12-2 p.o #2.: | | | | | | |
| None | - | - | 2.8 | ND | 5.0 | 3.0 |
| Mitogen (10 ug/ml) | 1.1 1 | 0.9 | 0.7 | ND | ND | ND |
| HAV (11 ug/mL) | 1.0 | 0. 8 | 2.5 | ND | ND | ND |
| HAV (5. 5 ug/mL) | 1.3 | 0. 8 | 3.1 | ND | ND | ND |
| HAV (2.8 ug/mL) | 1.0 | 1.1 | 3.0 | ND | ND | ND |
| HAV (1.4 ug/mL) | 2.1 | 1.2 | 3.2 | ND | ND | ND |
| | | | | | | |
| T12-2 p.o #3.: | | | | | | |
| None | - | - | 2.3 | ND | 4.5 | 1.0 |
| Mitogen(10ug/ml) | 1.1 | 2.3 | 2.3 | ND | ND | ND |
| HAV (11 ug/mL) | 1.1 | 1.2 | 2.3 | ND | ND | ND |
| HAV (5.5 ug/mL) | 1.0 | 1.6 | 3.9 | ND | ND | ND |
| HAV (2.8 ug/mL) | 0.6 | 1.0 | 7.2 | ND | ND | ND |
| HAV (1.4 ug/mL) | 0.9 | 2.3 | 2.1 | ND | ND | ND |
| | | | | | | |
| T12-3 p.o. #1: | | | | | | |
| None | - | - | 2.6 | ND | 2.0 | 0 |
| Mitogen (10 ug/ml) | 1.5 | 1.6 | 2.4 | ND | ND | ND |
| HAV (11 ug/mL) | 0.9 | 1.8 | 2.6 | ND | ND | ND |
| HAV (5.5 ug/mL) | 1.1 | 1.2 | 2.0 | ND | ND | ND |
| HAV (2.8 ug/mL) | 0.5 | 1.0 | 1.9 | ND | ND | ND |
| HAV (1.4 ug/mL) | 0.5 | 1.3 | 1.8 | ND | ND | ND |
| | | | | | | |
| T12-3 p.o. #2: | | | | | | |
| None | - | - | 0.5 | ND | 4.3 | 2.8 |
| Mitogen (10 ug/ml) | 0.8 | 2.2 | 2.1 | ND | ND | ND |
| HAV (11 ug/mL) | 2.2 | 2.1 | 1.6 | ND | ND | ND |
| HAV (5.5 ug/mL) | 2.1 | 2.2 | 2.2 | ND | ND | ND |
| HAV (2.8 ug/mL) | 0.5 | 2.7 | 2.3 | ND | ND | ND |
| HAV (1.4 ug/mL) | 0.7 | 2.0 | 1.8 | ND | ND | ND |
| | | | | | | |
| T12-3 p.o. #3: | | | | | | |
| None | - | - | 2.0 | ND | 2.0 | 0.8 |
| Mitogen (10 ug/ml) | 1.1 | 1.6 | 1.5 | ND | ND | ND |
| HAV (11 ug/mL) | 1.6 | 1.6 | 2.1 | ND | ND | ND |
| HAV (5.5 ug/mL) | 1.7 | 1.1 | 2.0 | ND | ND | ND |
| HAV (2.8 ug/mL) | 1.2 | 1.2 | 2.1 | ND | ND | ND |
| HAV (1.4 ug/mL) | 1.0 | 1.3 | 2.1 | ND | ND | ND |

| | | | | | | |
|---|---|---|---|---|---|---|
| ^{a}Immunization protocol by animal. Animals were boosted (i.d.) with 27.5 ug of HAV protein (Microbix HAV antigen) 10-28 days before testing. ^{b}Stimulation index (SI) determined from ³H-TdR incorporation experiments: no. of cpm incorporated into cells in stimulated wells divided by the no. of cpm incorporated into cells in unstimulated (medium only) wells. Mitogen used was PHA. SI≥ 2.0 considered positive. ^{c}HAV-specific IgA S/N (signal:noise ratio) as determined using EIA by dividing A405 nm measured in wells coated with HAV antigen by A405nm measured in wells receiving coating buffer only. Mitogen used was PWM. S/N ≥ 2.0 considered positive. ^{d}No. of positive ELISpots: average no. of ELISpots counted in wells coated with HAV above averaged background of ELISpots counted in wells coated with FRhK4 antigen or coating buffer only. | | | | | | |

### Results of Experiments Following i.d. Boosting of Immunity to HAV: Negative Controls (not boosted).

| Immunization | IgA Stimulation Index (SI)^{b} | | Specific IGA (S/N)^{c} | | No. of ELIspots (Mean^{d}) | |
|---|---|---|---|---|---|---|
| Stimulant^{a} | SPCL | PPC | SPCL | PPC | SPCL | PPC |
| Saline p.o.: | | | | | | |
| None | - | - | - | 1.6 | ND | |
| Mitogen (10 ug/ml) | 1.1 | 2.4 | 2.6 | ND | ND | ND |
| HAV (11 ug/mL) | 2.0 | 1.9 | 1.9 | ND | ND | ND |
| HAV(5.5ug/mL) | 1.1 | 1.5 | 2.2 | ND | ND | ND |
| HAV (2.8 ug/mL) | 0.3 | 0.9 | 1.9 | ND | ND | ND |
| HAV (1.4 ug/mL) | 0.9 | 1.8 | 1.0 | ND | ND | ND |
| | | | | | | |
| Saline i.r. #1: | | | | | | |
| None | - | - | - | 2.0 | ND | |
| Mitogen (10 ug/ml) | 1.3 | 1.5 | 2.4 | ND | ND | ND |
| HAV (11 ug/mL) | 1.4 | 1.0 | 2.3 | ND | ND | ND |
| HAV (5.5 ug/mL) | 1.7 | 1.1 | 3.0 | ND | ND | ND |
| HAV(2.8 ug/mL) | 1.1 | 1.1 | 3.6 | ND | ND | ND |
| HAV (1.4 ug/mL) | 0.8 | 1.4 | 2.1 | ND | ND | ND |
| | | | | | | |
| Saline i.r #2: | | | | | | |
| None | - | - | - | 2.2 | ND | |
| Mitogen (10 ug/ml) | 0.9 | 1.0 | 2.7 | ND | ND | ND |
| HAV (11 ug/mL) | 1.4 | 0.8 | 2.1 | ND | ND | ND |
| HAV (5.5 ug/mL) | 1.2 | 1.1 | 2.1 | ND | ND | ND |
| HAV (2.8 ug/mL) | 0.9 | 1.0 | 2.0 | ND | ND | ND |
| HAV (1.4 ug/mL) | 1.1 | 1.1 | 1.8 | ND | ND | ND |

### SUMMARY OF RESULTS

The hepatitis A viral (HAV) antigen (purchased from Microbix as a semi-purified preparation grown in FRhK4 monkey kidney cells) is immunogenic in mice when delivered by:
the intraperitoneal (i.p.) route emulsified with incomplete Freund's adjuvant (IFA); intrarectally (i.r.) by direct application or orally (p.o.) by passive feeding.

HAV antigen given by i.p., i.r., or p.o. routes generates HAV-responsive (memory) lymphocytes in both the spleen cell (SPLC) and Peyer's patch cell (PPC) populations that can be detected by *in vitro* proliferation assays. In mice immunized via the mucosal (i.r. or p.o.) routes the proliferative response appears to be more prevalent and more vigorous in the PPC population than in the SPLC population.

HAV antigen given by the i.p., i.r., or p.o. routes also gives rise to HAV-specific IgA forming cells in both the SPLC and PPC populations which can be detected by ELISpot assays either directly (after immunization) or indirectly (after *in vitro* boosting in bulk culture). However, cells forming IgA reactive with FRhK4 antigens as well as other assay constituents are also detectable by ELISpot assays. Therefore, results have been expressed as the net number of positive (HAV-specific) spots after subtraction of this background reactivity.

HAV-specific cells present in the SPLC and PPC populations can be induced to synthesize and secrete HAV-specific IgA detectable by direct enzyme immunoassay (EIA) after *in vitro* stimulation in bulk culture with pokeweed mitogen (PWM) or HAV antigen at various concentrations.

Two transgenic tomato vaccines given by the p.o. route also appeared to be immunogenic in that they provoked immunologic memory lymphocytes in the spleen and Peyer's patches that were detectable by proliferation assays.

### SECTION 4: FUTURE IMPLEMENTATIONS

The previously described transgenic plant vaccine contains non-infectious viral particles. These viral particles may also optionally be used to package appropriate genes engineered into the HAV genome, or another viral genome, which could subsequently be engineered into tomato and/or other plants. In this case, eating such transgenic tomatoes or other plant material, or otherwise applying the transgenes orally and/or rectally, would permit the transgenes to be specifically targeted to the liver by using HAV virus, or to other locations in the body, such as the bone marrow or the nervous system, by using other viruses. Without wishing to be limited to a single hypothesis, it is hypothesized that following ingestion of the HAV-containing transgenic tomatoes, the immune system should recognize the viral nucleocapsid proteins following the presentation of viral peptides by the MHC type I molecules in M type cells or other APC's in the gut epithelium. Vaccines for polioviruses (another member of the picornavirus family) and other viral agents could be developed using the same principle, namely, employing the natural viral tropism to the epithelial cells covering the gastrointestinal tract. These vaccines could be safer and/or easier to administer than existing vaccines, as well as being relatively simple and inexpensive to produce. The success of HAV particle production in transgenic plants would be the proof of principle for the production of other orally consumed viral and bacterial vaccines.

The efficient viral uptake by the gastrointestinal system could also be applied for targeting of transgenes to specific organs. The natural tropism of HAV is to the liver, probably through the expression of a viral receptor on hepatocyte cell membrane. Encapsidating the engineered HAV genome containing the desired gene in HAV nucleocapsid particles would enable targeting of genes to the liver. Similar targeting of genes to the bone marrow or the nervous system is potentially feasible using a similar strategy employing non-envelope viruses (or other suitable types of viruses) with a natural tropism for specific organs.

While the invention has been described with respect to a limited number of embodiments, it will be appreciated that many variations, modifications and other applications of the invention may be made.

## Claims

1. A vaccine against an encapsidated virus for administration to a subject, comprising: an edible material from a plant expressing a recombinant viral capsid or virus-like particle of said virus, wherein said recombinant viral capsid or virus-like particle comprises a plurality of different viral proteins co-expressed under control of the same at least one regulatory element, and is selected capable of producing a mucosal immune response in said subject.

2. The vaccine of claim 1, wherein said plant is transgenic for genes encoding said viral capsid or virus-like particle, such that said genes are stably inserted into a genome of said plant.

3. The vaccine of claim 1, wherein said encapsidated virus is a HAV (Hepatitis A virus), such that said genes are HAV genes.

4. The vaccine of claim 3, wherein said HAV genes are selected from the group consisting of VP1, VP2 and VP3.

5. The use of claim 3, wherein said HAV genes are VP1, VP2 and VP3.

6. The vaccine of claim 1, wherein said at least one regulatory element comprises a promoter and/or a translation enhancer.

7. The vaccine of claim 6, wherein said at least one regulatory element comprises a 35SCaMV promoter and an Omega (TMV) translation-enhancer box.

8. The vaccine of claim 1, wherein said plant is a tomato plant.

9. The vaccine of claim 1, formulated for administration of the vaccine by being eaten by the subject.

10. The vaccine of claim 1, further comprising a carrier adapted to administration of the vaccine to the gastrointestinal mucosa of a subject.

11. The vaccine of claim 10, wherein said gastrointestinal mucosa is a rectal mucosa of a subject.

12. The vaccine of claim 11, wherein said vaccine is formulated in a form of a suppository.

13. The vaccine of claim 1, wherein the subject is a human being.

14. Use of the vaccine of any of claims 1 to 13 for the manufacture of a medicament for immunizing against an encapsidated virus.
